# EUROPEAN PATENT SPECIFICATION

(11) **EP 0 478 850 B1**
(45) Date of publication and mention of the grant of the patent: **14.09.1994**
(21) Application number: 90202645.9
(22) Date of filing: 04.10.1990
(51) Int. Cl.: C07C 45/58, C07C 47/19

(54) **Process for the preparation of beta-hydroxy aldehydes**
Verfahren zur Herstellung von beta-Hydroxyaldehyden
Procédé pour la préparation de bêta-hydroxy aldéhydes

(43) Date of publication of application: 08.04.1992
(73) Proprietor: SHELL INTERNATIONALE RESEARCH MAATSCHAPPIJ B.V., 2596 HR Den Haag (NL)
(72) Inventor: Drent, Eit, NL-1031 CM Amsterdam (NL)

(56) References cited:
- EP-A- 0 257 967
- US-A- 3 463 819
- CHEMICAL ABSTRACTS, vol. 71, 1969, page 309, abstract no. 90820k, Columbus,Ohio, US; & JP-A-69 12 403
- BULLETIN OF THE CHEMICAL SOCIETY OF JAPAN, vol. 37, 1964, pages 677-679; C.YOKOKAWA et al.: "Studies of the organic reactions of metal carbonyl. III. The hydroformyletion of olefin oxides"

## Description

The present invention relates to a process for the preparation of β-hydroxyaldehydes.

β-Hydroxyaldehydes are useful as starting materials for the preparation of a wide variety of products, for example dicarboxylic acids and derivatives thereof. It is known from US patent No. 3,463,819 and European patent application publication No. EP-A2-0257967 that they may be obtained from epoxides in small quantities by reaction with carbon monoxide and hydrogen in the presence of a phosphine-modified catalyst containing either cobalt or rhodium.

Surprisingly, it has now been found that β-hydroxyaldehydes may be obtained from epoxides with high conversion and selectivity using a phosphine-modified catalyst containing cobalt and rhodium.

The invention therefore provides a process for the preparation of β-hydroxyaldehydes, which comprises reacting an epoxide with carbon monoxide and hydrogen in the presence of a catalyst comprising a combination of a rhodium compound and a cobalt compound, modified with a tertiary phosphine.

The epoxide employed in the process of the present invention may be an acyclic or cyclic epoxide. Acyclic epoxides are herein considered to be those epoxides wherein the carbon atoms of the epoxy moiety are not members of a carbocyclic ring. Preferred acyclic epoxides have a vicinal or 1,2-epoxy group and are based on saturated hydrocarbons having up to 10 carbon atoms wherein the hydrocarbon entity may have an acyclic or cyclic configuration. Examples of a cyclic epoxides include ethylene oxide, propylene oxide, isobutylene oxide, 1,2-epoxyoctane, 3-cyclohexyl-1,2-epoxypropane, 3,4-epoxynonane, 1,2-epoxy-2,4,4-trimethylhexane and 1,2-epoxydecane. Cyclic epoxides are herein considered to be those epoxides wherein the carbon atoms of the epoxy moiety are also members of a carbocyclic ring. Examples of cyclic epoxides include cyclohexene oxide, cyclopentene oxide, cyclooctene oxide, 1,2-epoxy-4-methylcyclohexane, 4,5-dimethyl-1,2-epoxycyclohexane, 2,3-epoxy-decahydronaphthalene and 1,2-epoxy-4-propylcyclohexane.

More preferred are epoxides having a 1,2-epoxy group and containing up to 6 carbon atoms, ethylene oxide and propylene oxide being especially preferred.

Rhodium compounds which may conveniently be employed in the catalyst, include rhodium oxides, rhodium halogenides, such as RhCl₃, RhBr₃ and RhI₃, rhodium chlorocarbonyl (RhCl(CO)₂)₂), rhodium nitrate, rhodium acetylacetonate (Rh(acac)₃) and rhodium carbonyl acetylacetonate (Rh(CO)₂(acac)), complexes of rhodium with tertiary phosphines, and mixtures thereof. Rh(CO)₂(acac) is a preferred rhodium compound.

Cobalt compounds which may conveniently be used in the catalyst for the process of the present invention in combination with the hereinbefore mentioned rhodium compounds, include cobalt salts of aliphatic acids such as cobalt acetate, cobalt formate, cobalt butyrate, cobalt carbonate, cobalt octanoate, cobalt oleate as well as dicobalt octacarbonyl (Co₂(CO)₈), complexes of cobalt and tertiary phosphine, and mixtures thereof. Co₂(CO)₈ is a preferred cobalt compound.

In addition to the hereinbefore mentioned rhodium compound and cobalt compound the catalyst also includes a tertiary phosphine. Such a phosphine generally has a stabilising function in the catalyst.

The tertiary phosphine is preferably a monodentate tertiary phosphine; that is a tertiary phosphine having a single phosphorus atom as the sole complexing site. This class of tertiary phosphines, hereinafter referred to as tertiary mono-phosphines, preferably has from 3 to 36 carbon atoms. Preferably at least one phosphorus substituent is aliphatic, and each phosphorus substituent is a hydrocarbon substituent , i.e., contains only atoms of carbon and hydrogen, and is free from aliphatic unsaturation. More preferably, each phosphorus substituent is aliphatic. Such phosphines are termed aliphatic tertiary monophosphines herein.

A preferred class of tertiary mono-phosphines may be represented by the formula RRRP wherein each R independently is monovalent hydrocarbon of up to 20 carbon atoms, preferably up to 12, and is free from aliphatic unsaturation, with the proviso that two R groups may together form a divalent hydrocarbon moiety of up to 12 carbon atoms. Of course, at least one phosphorus substituent R is aliphatic as is previously stated. Each group R, when monovalent, is therefore alkyl, cycloalkyl or aryl of up to 12 carbon atoms, preferably of up to 6 carbon atoms, and is illustrated by alkyl groups such as methyl, ethyl, butyl, isobutyl, 2-ethylhexyl, octyl, benzyl, β-phenylethyl and dodecyl; by cycloalkyl groups such as cyclopentyl, cyclohexyl, cyclooctyl, 2,3-diethylcyclopentyl, 4-butylcyclohexyl, 2,4,5-trimethylcyclohexyl and 3-butylcyclooctyl; and by aryl groups such as phenyl, tolyl, xylyl, o-phenylphenyl, p-tert-butylphenyl, 2,4-diethylphenyl and m-cyclohexylphenyl.

When two R groups together form a divalent hydrocarbon moiety, the hydrocarbon moiety is preferably an alkylidene or cycloalkylidene group.

One preferred class of phosphines of formula RRRP is that wherein each R is a straight-chain primary alkyl group, e.g. tri-n-butylphosphine. Another preferred class of phosphines of formula RRRP is that wherein two R groups together form a divalent hydrocarbon moiety. Examples of such phosphines are 1-ethylphospholidine, 1-phenylphospholidine, 1-phenylphosphorinane, 1-butyl-phosphorinane, 4,4-dimethyl-1-phenylphosphorinane, 1-phenylphosphepane, 1-ethylphosphepane, 3.6-dimethyl-1-phenylphosphepane, 9-phenyl-9-phosphabicyclo[4.2.1]nonane, 9-phenyl-9-phosphabicyclo[3.3.1]nonane, and 9-butyl-9-phosphabicyclo[4.2.1]nonane.

The tertiary phosphine may alternatively be a bidentate ligand, i.e., a tertiary di-phosphine. Preferred tertiary di-phosphines are represented by the formula RRP-R'-PRR wherein R has the previously stated meanings, and R' is a divalent, saturated, aliphatic hydrocarbon moiety, preferably an α,ω-alkylene of from 2 to 3 carbon atoms. Illustrative di-phosphines of this class include 1,2-bis(diphenylphosphino)ethane, 1,2-bis(dibutylphosphino)ethane, 1,3-bis(dimethylphosphino)propane, 1,3-bis(dihexylphosphino)propane, 1,2-bis(ditolylphosphino)ethane, 1,3-bis(phenylpropylphosphino)propane, 1-(dibutylphosphino)-3-(diphenylphosphino)propane and 1-(dioctylphosphino)-2-(dibutylphosphino)propane.

Preferred tertiary phosphines in the process of the present invention are aliphatic tertiary mono-phosphines.

In the process according to the invention, the rhodium compound and the cobalt compound are preferably employed in a ratio which corresponds with a ratio of gram atoms rhodium to gram atoms cobalt in the range of from 0.5:1 to 10:1, more preferably in a range 1:1 to 10:1, even more preferably in the range of from 1:1 to 5:1. The tertiary phosphine is preferably employed in an amount which corresponds with a ratio of gram atoms of phosphorus of the phosphine to gram atoms of rhodium and cobalt in the range of from 0.5:1 to 10:1, more preferably 1:1 to 4:1.

The catalyst as described hereinbefore is believed to be novel. Hence the present invention also relates to a catalyst composition comprising a tertiary phosphine, a rhodium compound and a cobalt compound, wherein the ratio of gram atoms of rhodium to gram atoms cobalt is in the range of from 1:1 to 10:1 and the ratio of gram atoms of phosphorus of the phosphine to gram atoms of rhodium and cobalt is in the range of from 1:1 to 4:1.

The process according to the invention may be effected in the presence of a minor amounts of a weakly acidic compound, preferably an acid having a pKa (measured at 18 °C in aqueous solution) > 2. Examples of weakly acidic compounds include phenylphosphonic acid, 2,4,5-trimethylbenzoic acid and hydroquinone.

The catalyst may conveniently be obtained by contacting the catalyst components under stirring in the ratio as specified hereinbefore, in the liquid reaction medium in the reactor. When desired the catalyst may be charged into the reactor as a preformed material obtained by contacting said components e.g. in a suitable inert solvent.

Although the preparation of the β-hydroxyaldehydes may conveniently be conducted by charging the catalyst components or the preformed catalyst to the reactor containing the epoxide, it is preferred to conduct the process of the present invention in a liquid phase which in addition to the epoxide contains an inert solvent. Although a wide variety of solvents may be used which are inert to the reactants and the catalyst and which are moreover liquid at the reaction temperature it is preferred to employ a non-hydroxylic, substantially anhydrous solvent which is free from aliphatic carbon-carbon unsaturation and contains no atoms other than carbon, hydrogen and oxygen. Illustrative of suitable solvents are hydrocarbons, particularly aromatic hydrocarbons of up to 10 carbon atoms such as benzene, toluene, xylene, ethylbenzene, and butylbenzene; ketones, particularly aliphatic ketones, i.e., alkanones, of up to 10 carbon atoms such as acetone, methyl ethyl ketone, methyl isobutyl ketone, ethyl hexyl ketone and dibutyl ketone; esters of up to 10 carbon atoms, particularly lower alkyl esters of carboxylic acids which acids are aliphatic or aromatic carboxylic acids having one or more carboxyl groups, preferably from 1 to 2, such as ethyl acetate, methyl propionate, propyl butyrate, methyl benzoate, diethyl glutarate, diethyl phthalate and dimethyl terephthalate; and ethers of up to 10 carbon atoms and up to 4 ether oxygen atoms, which ethers are cyclic or acylic ethers and which are preferably aliphatic ethers, e.g., diethyl ether, diisopropyl ether, dibutyl ether, ethyl hexyl ether, methyl octyl ether, dimethoxyethane, diethylene glycol dimethyl ether, diethylene glycol diethyl ether, glycerol trimethyl ether, 1,2,6-trimethoxyhexane, tetrahydrofuran, 1,4-dioxane, 1,3-dioxane, 1,3-dioxolane and 2,4-dimethyl-1,3-dioxane.

Preferred solvents to be used in the process of the present invention are glycol ethers such as diethylene glycol dimethyl ether and diethylene glycol diethyl ether.

The amount of solvent to be employed is not critical. Typical molar ratios of solvent to epoxide will be in the range of from 2:1 to 150:1. The optimum ratio of epoxide to phosphine-modified rhodium/cobalt-based catalyst will partly be determined by the nature of the individual catalyst components as well as by the nature of the epoxide. The molar ratio of epoxide to rhodium of the catalyst, may vary over wide ranges and will preferably be in the range of from 10:1 to 10⁶:1 and more preferably in the range of from 10²:1 to 10⁶:1.

The molar ratio of carbon monoxide to hydrogen most suitably employed in the process according to the invention is in the range of from 6:1 to 1:4, with best results being obtained when ratios of from 3:1 to 1:2 are utilised. No special precautions need to be taken with regard to the carbon monoxide and hydrogen, and commercial grades of these reactants are satisfactory. The carbon monoxide and hydrogen are suitably employed as separate materials although it is frequently advantageous to employ commercial mixtures of these materials, e.g., synthesis gas.

The process is conducted, in one modification, by charging the epoxide, catalyst and solvent to an autoclave or similar pressure reactor and introducing the hydrogen and carbon monoxide. Alternatively, the process is conducted in a continuous manner by contacting the reactants and catalyst during passage through a reactor which is typically tubular in form. For best results the process is conducted under conditions of elevated temperature and pressure. Reaction temperatures in the range of from 50 °C to 250 °C are satisfactory, with temperatures in the range of from 80 °C to 150 °C being preferred. In one method of effecting reaction, the reactants and catalysts are initially contacted at a comparatively low reaction temperature e.g. from 50 - 70 °C and the temperature is subsequently raised gradually or in stages during the course of the reaction to e.g. a desired end temperature. The reaction pressure may vary over a wide range and will generally be in the range of from 5 to 150 bar, although higher and lower pressures are not excluded. Frequently the upper limit of the reaction pressure will be dictated by the technical specification of the equipment employed, rather than by the actual reaction or reactants.

The invention will be further illustrated by the following Examples and Illustrative Embodiments for which the following information is provided.
Product identification and analysis. The reaction products were qualitatively analysed with the aid of Gas liquid chromatographic separation in combination with NMR and/or mass spectrography. Quantitative analysis was conducted via G.L.C.

### Example I

0.3 mmol of Rh(CO)₂(acac), 0.1 mmol Co₂(CO)₈ and 0.5 mmol of tri-cyclohexyl phosphine were charged into a reactor containing 50 ml of diethylene glycol dimethyl ether. After a number of pressurizing depressurizing cycles with carbon monoxide to remove air, 5 ml of ethylene oxide was introduced and then the reactor was pressurized with a 40:14 molar mixture of carbon monoxide and hydrogen to a pressure of 54 bar. The temperature of the reactor was then raised to 90 °C. At the end of the reaction (10 hr) 50% of the epoxide had been converted. The reaction product comprised 3-hydroxy-propanal-1 (90%) and acetaldehyde (5%) as the major components.

### Illustrative Embodiment I

0.3 mmol of Rh(CO)₂(acac), 0.1 mmol Co₂(CO)₈ and 0.8 mmol of tributylphosphine are charged into a reactor containing 50 ml of diethylene glycol dimethyl ether. After a number of pressurizing depressurizing cycles with carbon monoxide to remove air, 5 ml of ethylene oxide is introduced and then the reactor is pressurized with a 2:1 molar mixture of carbon monoxide and hydrogen to a pressure of 60 bar. The temperature of the reactor is then raised to 100 °C. Similar results to those of Example I may be obtained.

### Illustrative Embodiment II

0.3 mmol of Rh(CO)₂(acac), 0.1 mmol Co₂(CO)₈ and 0.8 mmol triisopropylphosphine are charged into a reactor containing 10 ml of propylene oxide and 50 ml of diethylene glycol dimethyl ether. After a number of pressurizing depressurizing cycles with carbon monoxide to remove air, the reactor is pressurized with a 2:1 molar mixture of carbon monoxide and hydrogen to a pressure of 60 bar. The temperature of the reactor is then raised to 110 °C. Similar results to those of Example I may be obtained.

### Illustrative Embodiment III

0.2 mmol of Rh(CO)₂(acac), 0.05 mmol Co₂(CO)₈ and 0.5 mmol trioctylphosphine are charged to a reactor containing 10 ml propylene oxide and 50 ml of diethylene glycol dimethyl ether. After a number of pressurizing depressurizing cycles with carbon monoxide to remove air, the reactor is pressurized with a 2:1 molar mixture of carbon monoxide and hydrogen to a pressure of 60 bar. The temperature of the reactor is then raised to 110 °C. Similar results to those of Example I may be obtained.

From the results of comparative experiments, it would be expected that the addition of the rhodium catalyst to the cobalt catalyst would have little or no effect, because the rhodium catalyst is much less active than the cobalt catalyst. Surprisingly, however, the combined catalyst has substantially greater selectivity.

## Claims

1. A process for the preparation of β-hydroxyaldehydes, which comprises reacting an epoxide with carbon monoxide and hydrogen in the presence of a catalyst comprising a combination of a rhodium compound and a cobalt compound, modified with a tertiary phosphine.

2. A process as claimed in claim 1, wherein the rhodium compound and cobalt compound are employed in a ratio which corresponds with a ratio of gram atoms of rhodium to gram atoms of cobalt in the range of from 1:1 to 10:1.

3. A process as claimed in claim 1 or claim 2, wherein the tertiary phosphine is employed in an amount which corresponds with a molar ratio of gram atoms of phosphorus of the phosphine to gram atoms of rhodium and cobalt in the range of from 1:1 to 4:1.

4. A process as claimed in any one of claims 1-3, wherein the rhodium compound is a rhodium oxide, a rhodium halogenide, rhodium chlorocarbonyl, rhodium nitrate, rhodium acetylacetonate, rhodium carbonyl acetylacetonate, a complex of rhodium with a tertiary phosphine or a mixture thereof, and the cobalt compound is a cobalt salt of an aliphatic acid, dicobalt octacarbonyl, a complex of cobalt with a tertiary phosphine, or a mixture thereof.

5. A process as claimed in any one of claims 1-4, wherein the tertiary phosphine is an aliphatic tertiary monophosphine.

6. A process as claimed in any one of claims 1-5, wherein a glycol ether is present as a solvent.

7. A process as claimed in any one of claims 1-6, wherein the temperature is in the range of from 80-150 °C, and the pressure is in the range of from 5-150 bar.

8. A process as claimed in any one of claims 1-7, wherein the epoxide is ethylene oxide or propylene oxide.

9. A catalyst composition comprising a tertiary phosphine, a rhodium compound and a cobalt compound wherein the ratio of gram atoms of rhodium to gram atoms of cobalt is in the range of from 1:1 to 10:1 and the ratio of gram atoms of phosphorus of the phosphine to gram atoms of rhodium and cobalt is in the range of from 1:1 to 4:1.

## Patentansprüche

1. Ein Verfahren zur Herstellung von β-Hydroxyaldehyden, welches die Umsetzung eines Epoxids mit Kohlenmonoxid und Wasserstoff in Gegenwart eines Katalysators, der eine Kombination aus einer Rhodiumverbindung und einer Kobaltverbindung umfaßt und mit einem tertiären Phosphin modifiziert ist, umfaßt.

2. Ein Verfahren, wie in Anspruch 1 beansprucht, in welchem die Rhodiumverbindung und die Kobaltverbindung in einem Verhältnis eingesetzt werden, das einem Verhältnis der Grammatome Rhodium zu den Grammatomen Kobalt im Bereich von 1:1 bis 10:1 entspricht.

3. Ein Verfahren, wie in Anspruch 1 oder 2 beansprucht, in welchem das tertiäre Phosphin in einer Menge eingesetzt wird, die einem Molverhältnis der Grammatome Phosphor des Phosphins zu den Grammatomen Rhodium und Kobalt im Bereich von 1:1 bis 4:1 entspricht.

4. Ein Verfahren, wie in einem der Ansprüche 1 bis 3 beansprucht, in welchem die Rhodiumverbindung ein Rhodiumoxid, Rhodiumhalogenid, Rhodiumchlorcarbonyl, Rhodiumnitrat, Rhodiumacetylacetonat, Rhodiumcarbonylacetylacetonat, ein Komplex von Rhodium mit einem tertiären Phosphin oder eine Mischung davon ist, und die Kobaltverbindung ein Kobaltsalz einer aliphatischen Säure, Dicobaltoctacarbonyl, ein Komplex von Kobalt mit einem tertiären Phosphin, oder eine Mischung davon ist.

5. Ein Verfahren, wie in einem der Ansprüche 1 bis 4 beansprucht, in welchem das tertiäre Phosphin ein aliphatisches tertiäres Monophosphin ist.

6. Ein Verfahren, wie in einem der Ansprüche 1 bis 5 beansprucht, in welchem ein Glykoläther als Lösungsmittel vorliegt.

7. Ein Verfahren, wie in einem der Ansprüche 1 bis 6 beansprucht, in welchem die Temperatur im Bereich von 80-150°C und der Druck im Bereich von 5 bis 150 Bar liegt.

8. Ein Verfahren, wie in einem der Ansprüche 1 bis 7 beansprucht, in welchem das Epoxid Ethylenoxid oder Propylenoxid ist.

9. Eine Katalysatorzusammensetzung, umfassend ein tertiäres Phosphin, eine Rhodiumverbindung und eine Kobaltverbindung, in welcher das Verhältnis der Grammatome Rhodium zu den Grammatomen Kobalt im Bereich von 1:1 bis 10:1 und das Verhältnis der Grammatome Phosphor des Phosphins zu den Grammatomen Rhodium und Kobalt im Bereich von 1:1 bis 4:1 liegt.

## Revendications

1. Procédé de préparation de β-hydroxyaldéhydes, qui comprend la réaction d'un époxyde avec du monoxyde de carbone et de l'hydrogène, en présence d'un catalyseur comprenant une combinaison d'un composé de rhodium et d'un composé de cobalt, modifié par une phosphine tertiaire.

2. Procédé selon la revendication 1, dans lequel le composé de rhodium et le composé de cobalt sont employés dans un rapport qui correspond à un rapport des atome-grammes de rhodium aux atome-grammes de cobalt, dans la gamme de 1 : 1 à 10 : 1.

3. Procédé selon la revendication 1 ou la revendication 2, dans lequel la phosphine tertiaire est employée en une quantité correspondant à un rapport molaire des atome-grammes du phosphore de la phosphine aux atome-grammes du rhodium et du cobalt dans la gamme de 1 : 1 a 4 : 1.

4. Procédé selon l'une quelconque des revendications 1-3, dans lequel le composé de rhodium est un oxyde de rhodium, un halogénure de rhodium, le rhodium-chlorocarbonyle, le nitrate de rhodium, l'acétylacétonate de rhodium, l'acétylacétonate de rhodium-carbonyle, un complexe de rhodium avec une phosphine tertiaire ou un mélange de ceux-ci, et le composé de cobalt est un sel de cobalt d'un acide aliphatique, le dicobalt-octacarbonyle, un complexe de cobalt et de phosphine tertiaire, ou un mélange de ceux-ci.

5. Procédé selon l'une quelconque des revendications 1-4, dans lequel la phosphine tertiaire est une mono-phosphine tertiaire aliphatique.

6. Procédé selon l'une quelconque des revendications 1-5, dans lequel un éther de glycol est présent comme solvant.

7. Procédé selon l'une quelconque des revendications 1-6, dans lequel la température est dans la gamme de 80-150°C et la pression est dans la gamme de 5-150 bars.

8. Procédé selon l'une quelconque des revendications 1-7, dans lequel l'époxyde est l'oxyde d'éthylène ou l'oxyde de propylène.

9. Composition de catalyseur comprenant une phosphine tertiaire, un composé de rhodium et un composé de cobalt, dans laquelle le rapport des atome-grammes de rhodium aux atome-grammes de cobalt est dans la gamme de 1 : 1 à 10 : 1 et le rapport des atome-grammes du phosphore de la phosphine aux atome-grammes du rhodium et du cobalt dans la gamme de 1 : 1 à 4 : 1.
